Europäisches Patentamt

European Patent Office

Office européen des brevets

(11) Publication number: **0 200 430**
**B1**

# EUROPEAN PATENT SPECIFICATION

(45) Date of publication of patent specification: **04.04.90**

(51) Int. Cl.⁵: **A 61 M 25/00**

(21) Application number: **86302895.7**

(22) Date of filing: **17.04.86**

(54) Torsional guide wire with attenuated diameter.

(30) Priority: **18.04.85 US 724624**

(43) Date of publication of application:
**05.11.86 Bulletin 86/45**

(45) Publication of the grant of the patent:
**04.04.90 Bulletin 90/14**

(84) Designated Contracting States:
**CH DE FR GB LI**

(56) References cited:
**EP-A-0 141 006**
**DE-A- 522 204**
**US-A-3 789 841**
**US-A-4 020 829**

(73) Proprietor: **ADVANCED CARDIOVASCULAR
SYSTEMS, INC.**
**1395 Charleston Road**
**Mountain View, CA 94039-7101 (US)**

(72) Inventor: **Morrison, David W.**
**5509 Willowbrook Drive**
**San Jose California 95118 (US)**

(74) Representative: **Bizley, Richard Edward et al
BOULT, WADE & TENNANT 27 Furnival Street
London EC4A 1PQ (GB)**

Courier Press, Leamington Spa, England.

# Description

This invention relates to a guide wire for use with catheters.

Guide wires heretofore have been provided. However, guide wires which make it possible to penetrate small blood vessels with adequate proximal strength for catheter tracking capabilities as, for example, in the kidney and which still permit high torsional capabilities have not been available. There is therefore a need for a torsional, attenuating diameter guide wire.

Document US-A-3789841 (Antoshkiw) discloses a guide wire comprising an elongate core element having proximal and distal ends and having a cross-sectional area decreasing in a direction towards the distal end, and a coil carried by, surrounding and secured to the core element, the coil having proximal and distal ends.

The elongate core element has a portion of uniform thickness covered by a plastics jacket. A single coil of uniform diameter is soldered to the core adjacent to the distal end of the jacket, extends over the tapered portion of the core and is connected to the distal end of the core to form a rounded tip.

The present invention is characterised in that the guide wire has a progressively attenuated diameter, the coil has a diameter which decreases in a direction towards the distal end, and that the coil is formed of a wire which has a diameter which decreases from one end to the other end and which is wound in a helix so that the larger diameter portion of the wire begins in a region closer to the proximal end and the small diameter portion of the wire ends in a region closer to the distal end.

An embodiment of the invention is now described by way of example and with reference to the accompanying drawings, in which:

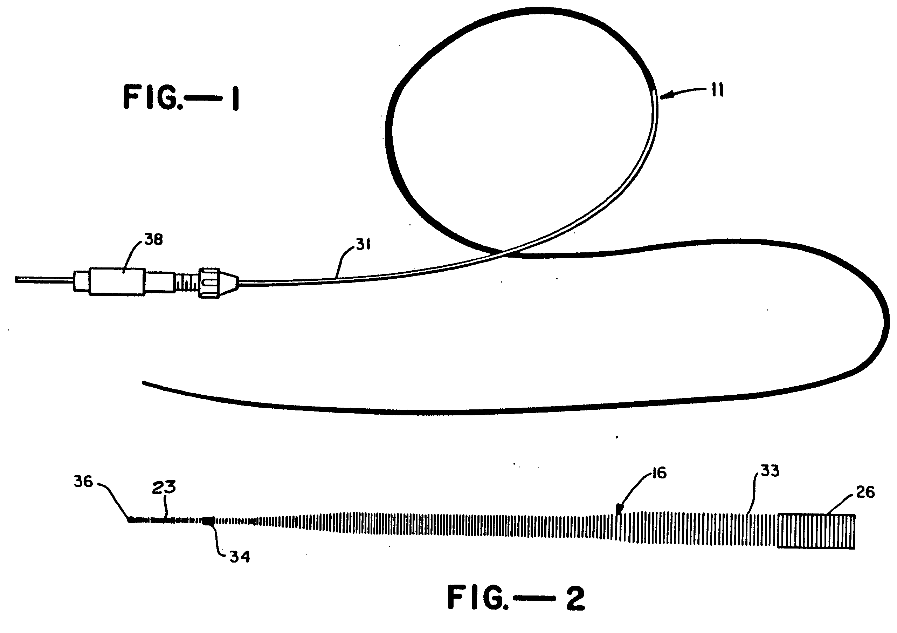

Figure 1 is a side elevational view of a torsional guide wire with a progressively attenuated diameter;

Figure 2 is an enlarged view of the distal extremity of the guide wire shown in Figure 1;

Figure 3 is a side elevational view of the core utilized in the guide wire shown in Figure 1;

Figure 4 is a side elevational view of the wire utilized for making the tapered coil for the guide wire in Figure 1;

Figure 5 is a side elevational view of the winding mandrel for the tapered coil of Figure 6;

Figure 6 is a side elevational view of the tapered coil made from winding the wire of Figure 4 onto the mandrel of Figure 5;

Figure 7 is a cross sectional view of the platinum coil tip utilized in the guide wire shown in Figure 1;

Figure 8 is a cross sectional view of the proximal coil utilized in the guide wire shown in Figure 1; and

Figure 9 is a side elevational view of the plastic tubing utilized in the guide wire shown in Figure 1.

As illustrated and in general, the torsional guide wire with a progressively attenuated diameter is comprised of an elongate core element having proximal and distal ends and having a cross sectional area decreasing in a direction towards the distal end. It is also comprised of a coil which is carried by and secured to the core element and extends over the distal extremity of the core element. The coil has proximal and distal ends and has a diameter which decreases in a direction towards the distal end. The coil is formed of wire which has a diameter which decreases from one end to the other end and is wound in a helix so that the larger diameter portion of the wire begins in a region closer to the proximal end and the smaller diameter portion of the wire ends in a region closer to the distal end.

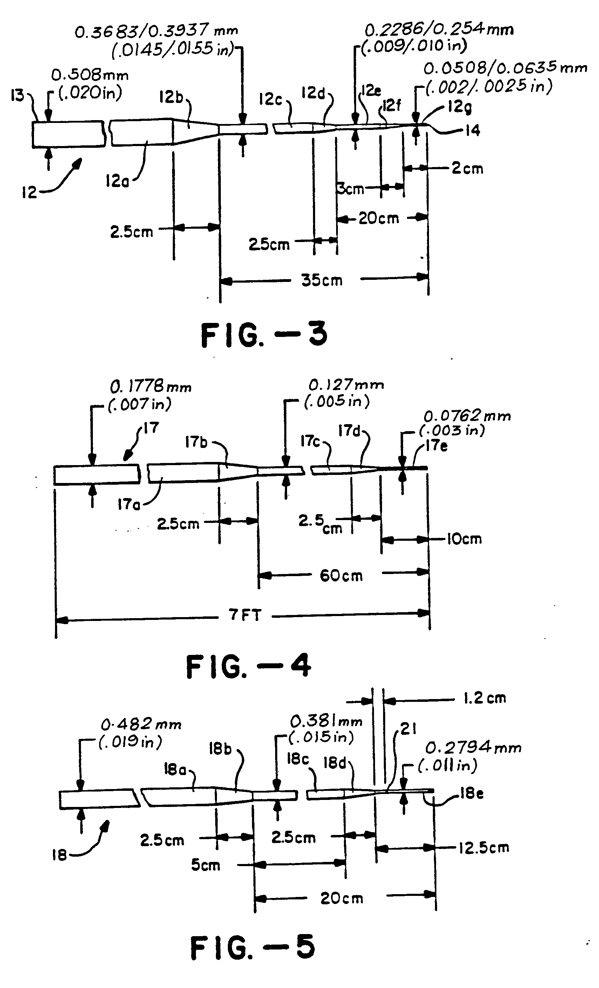

In the drawings, an attenuating diameter guide wire 11 comprises a core element 12 and a coil 16. The core element 12 is shown in detail in Figure 3 and is formed of a suitable material such as stainless steel and has a suitable diameter such as from 0.381 to 0.635mm (0.015 to 0.025 inch) and preferably a diameter of 0.508mm (0.020 inch), plus or minus 7.62μm (0.0003 inch) and having a suitable length such as 2.134m (7 feet). The core element is provided with proximal and distal ends 13 and 14. The core element 12 is centerless ground to provide a core wire which has a cross sectional area decreasing in a direction towards the distal end. Thus as shown, the core element 12 is provided with a cylindrical portion 12a which extends over a major portion of the length of the core element. A tapered portion 12b adjoins the portion 12a and extends over a suitable distance such as 2.5cm and provides a taper, for example, from 0.508mm (0.020 inches) to 0.3683 to 0.3937mm (0.0145 to 0.0155 inches) and preferably a dimension of 0.381mm (0.015 inches). The remaining length of the core element 12c is centerless ground to a dimension such as 0.381mm (0.015 inches) for a length of approximately 35cm. Thereafter, in a further grinding operation a transition region 12d is provided which over a length of approximately 2.5cm provides a transition from 0.381mm to 0.2286 to 0.254mm (0.015 inches to 0.009 to 0.010 inches). The following portion 12e of approximately 15cm length is centerless ground to the 0.254mm (0.010 inch) dimension. Thereafter an additional transitional region 12f is provided which provides a transition from 0.254mm to 0.0508 to 0.0635mm (0.010 inches to 0.002 to 0.0025 inches). This transition extends over approximately 3cm. The remaining distal extremity of the core wire has, for example, a length of approximately 2cm, is ground so that it has a diameter of 0.0508 to 0.0635mm (0.002 to 0.0025 inches). Thus it can be seen that a core element has been provided which has been carefully dimensioned by centerless grinding to provide a decreasing cross sectional area or a taper.

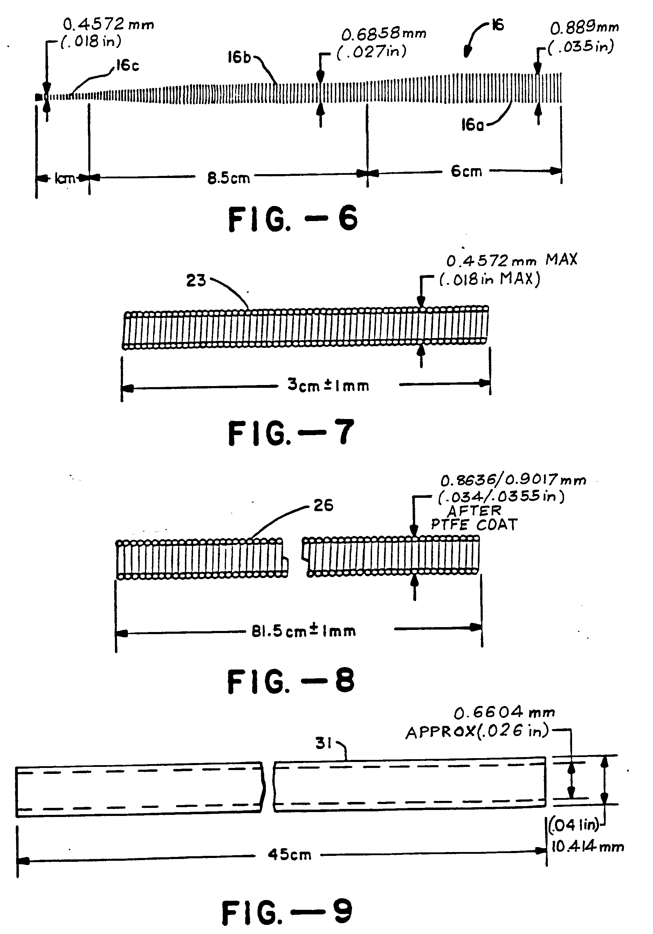

The coil 16 itself is shown in Figure 6. A wire 17 from which it is wound is shown in Figure 4. A mandrel 18 on which the wire 17 is wound to form the coil 16 is shown on Figure 5. The wire 17 is formed of a suitable material such as stainless steel and has a length of approximately 2.134m (7 feet) and a diameter of 0.1778mm (0.007 inches) plus or minus 5.08µm (0.0002 inches). A major portion of the wire 17 has an outer dimension of 0.1778mm (0.007 inches) but the remainder of the wire is centerless ground to provide a diameter decreasing towards the distal extremity of the wire. Thus in addition to the cylindrical portion 17a there is provided a transition portion 17b of a suitable length such as 2.5cm in which there is dimension change from 0.1778 to 0.127mm (0.007 to 0.005 inches). For a length of approximately 47.5cm from the distal extremity of the wire, a portion 17c of the wire has a diameter of 0.127mm (0.005 inches). At another portion 17d of the wire, another transition in diameter is made over a distance of approximately 2.5cm from 0.127 to 0.0762mm (0.005 to 0.003 inches). There is then provided a portion 17e having a length of approximately 10cm which has a diameter of 0.0762mm (0.003 inches).

The mandrel 18 on which the wire 17 is wound is shown in Figure 5 and is formed of a suitable material such as steel having a diameter of 0.4826mm (0.019 inches) extending over a portion 18a. A transition portion 18b is provided extending over 2.5cm in which the diameter is decreased from 0.4826 to 0.381mm (0.019 to 0.015 inches). Another portion 18c, 5cm long, is provided having a diameter of 0.381mm (0.015 inches) extending up to another transition portion 18d in which the diameter is decreased to 0.2794mm (0.011 inches) over a length of 2.5cm so that the distal extremity of the mandrel provided by the portion 18e has a diameter of 0.2794mm (0.011 inches) and a length of approximately 12.5 cm.

The coil or coil section 16 is then formed by taking the wire 17 and taking the end of the smallest diameter portion 17e and starting it on the mandrel at the point 21 about 1.2cm from the smaller end of portion 18d and progressively winding the wire onto the mandrel 18 in a helix in a direction towards the larger diameter end over a distance of approximately 15.5cm. Thus, the larger diameter portion of the wire begins in a region closer to the proximal end and the smaller diameter portion of the wire ends in a region closer to the distal end of the coil 16. After the winding has been completed, the coil or coil section 16 can be removed from the mandrel and trimmed to length. The proximal and distal ends of the coil 16 can be stretched apart slightly so that the ends can be screwed together with other coils or coil sections as hereinafter described.

A coil section 23 forms the tip of the guide wire 11 and is formed of a suitable wire such as a platinum alloy having a diameter of 0.0762mm (0.003 inches) plus or minus 5.08µm (0.0002 inches). This coil section is 3cm plus or minus 0.1cm long and has an outside diameter of approximately 0.4572mm (0.018 inches) maximum. The interior diameter corresponds to the diameter of the mandrel which is approximately 0.2794mm (0.011 inches). One end of the coil 23 is stretched so as it make it possible to screw into another coil end as hereinafter described.

The coil or coil section 16 after being wound and removed from the mandrel is formed so that it is provided with a portion 16a having a length of 6cm and an outside diameter of approximately 0.889mm (0.035 inches), another portion 16b having a length of 8.5cm and an outside diameter of approximately 0.6858mm (0.027 inches) and a distal extremity 16c having a length of 1cm and an outside diameter of approximately 0.4572mm (0.018 inches).

Another coil or coil section 26 forms the proximal coil. This coil is formed by winding a suitable material such as stainless steel wire having a diameter of 0.1778mm (0.007 inches) on the portion 18a of the mandrel 18 having a diameter of 0.4826mm (0.019 inches). The coil 26 is then coated with a suitable plastic material such as PTFE and is cut to a suitable length, as for example, 81.5cm with 1cm near the distal extremity being uncoated and stretched for a screw together brazed joint as hereinafter described. The outer diameter after coating is in the range 0.8636 to 0.9017mm (0.034 to 0.0355 inches).

The guide wire 11 also includes a length of shrink tubing 31 for example polyethylene having a suitable length as, for example, 45cm as shown in Figure 9 and having an outside diameter of approximately 10.41mm (0.41 inches) and an inside diameter of approximately 0.6604mm (0.026 inches).

With these fabricated components hereinbefore described, the guide wire may now be assembled. To start the assembly, the shrink tubing 31 is taken and is shrunk down onto the ground core element 12 approximately 100cm from the distal tip 14 of the core element 12. This can be accomplished in any suitable manner as, for example, by a batch process or by individual treatment of the shrink tubing with heat. The PTFE coated coil 26 is then placed on the core element 12 and bonded to the core element 12 at the point at which it is juxtaposed to the distal extremity of the shrink tubing 31 by the use of a suitable material such as cyanoacrylate. The coil or coil section 16 is then positioned on the core element 12 and its proximal extremity is threaded into the distal extremity of the PTFE coated coil 26 and thereafter a brazed joint 33 is formed to bond the juxtaposed ends of the coils 16 and 26 together. The screw together joint 33 with the brazing is shown in Figure 2. A gold alloy can be utilized to form the joint 33. The use of gold which is radiopaque permits the doctor to see where the 0.889mm (0.035 inch) diameter portion of the guide wire 11 starts by the use of a fluoroscope. The coil 16 when so positioned on the core element 12 has its larger diameter portion of the wire beginning in a region closer to the proximal end and its smaller diameter portion of the wire ending in a region closer to the distal end.

The platinum coil 23 is then taken and its proximal extremity is positioned on the core element 12 and threaded into the distal extremity of the coil or coil section 16. After they have been screwed together, a suitable solder can be utilized for forming a joint 34 between the juxtaposed ends of the coils 23 and 16 and also to bond them to the distal extremity of the core element 12. A tip 36 which has a rounded configuration is then provided on the distal extremity of the platinum coil 23.

After these assembly operations been completed, a torquer 38 of a conventional construction is mounted on the proximal extremity of the polyethylene shrink tubing 31. The torquer 38 firmly grasps the guide wire 11 and makes it possible for a physician by use of a hand to apply torque to the guide wire. The guide wire has a suitable overall length of 145cm.

The operation and use of the guide wire 11 is very similar to that for other guide wires. It, however, has numerous characteristics which are particularly adapted to particular types of operations as, for example, where it is desired to make deeper penetration into the kidney of the human body. It also has a very small diameter tip while still having a relatively large diameter proximal end to facilitate tracking of the catheter under the fluoroscope. The tapering of the coils or coil sections is particularly important and is made possible by the use of a tapered wire which is utilized for forming the tapered coil or coil sections. In addition, the screw together joints between the coil sections provides for transitions between the coil sections while still providing a smooth exterior circumferential surface.

It is apparent from the foregoing that there has been provided a guide wire which has a progressively attenuated diameter and which has good torsional capabilities. It also has a construction which enables it to be economically manufactured in quantity with great precision. Even though a tapered tip sub assembly has been provided in connection with a PTFE coated coil and polyethylene shrink tubing, there is a very smooth transition provided between the different materials utilized in the guide wire facilitating its use in connection with small vessels in humans.

## Claims

1. A guide wire comprising an elongate core element (12) having proximal and distal ends (13, 14) and having a cross-sectional area decreasing in a direction towards the distal end, and a coil (16) carried by, surrounding and secured to the core element (12), the coil (16) having proximal and distal ends, characterised in that the guide wire has a progressively attenuated diameter, the coil (16) has a diameter which decreases in a direction towards the distal end, and that the coil (16) is formed of a wire (17) which has a diameter which decreases from one end to the other end and which is wound in a helix so that the larger diameter portion of the wire begins in a region closer to the proximal end and the smaller diameter portion of the wire ends in a region closer to the distal end.

2. A guide wire as claimed in claim 1, comprising a second coil (23) formed of a radiopaque material having an end juxtaposed to the distal extremity of the first said coil (16).

3. A guide wire as claimed in claim 2, characterised in that the second coil (23) has a substantially uniform diameter and a rounded tip (36) carried at its distal extremity.

4. A guide wire as claimed in claim 2 or claim 3, characterised in the juxtaposed ends of the first and second coils (16, 23) are screwed together and are bonded to the core element (12).

5. A guide wire as claimed in any preceding claim, comprising a third coil (26) having proximal and distal ends carried by the core element (12) and having its distal end juxtaposed to the proximal end of the first said coil (16) and means (33) form a bond between the juxtaposed ends of the first and third coils and the core element.

6. A guide wire as claimed in claim 5, characterised in that the third coil (26) is covered with a plastic coating.

7. A guide wire as claimed in claim 5 or claim 6 together with polyethylene tubing (31) which is carried by the proximal extremity of the core element (12) and has its distal extremity juxtaposed to the proximal extremity of the third coil (26).

8. A guide wire as claimed in any preceding claim, characterised in that the elongate core element (12) has cylindrical portions of different diameters and tapered portions of different diameters adjoining the cylindrical portions.

9. A guide wire as claimed in any preceding claim, characterised in that the first said coil (16) is formed of a wire (17) having cylindrical portions of different diameters with tapered portions of different diameters adjoining the cylindrical portions.

## Patentansprüche

1. Führungsdraht, der ein längliches Kernelement (12), das proximale und distale Enden (13, 14) und einen Querschnittsbereich hat, der in Richtung zu dem distalen Ende kleiner wird, und eine Spirale (16) aufweist, die von dem Kernelement (12) getragen wird, dieses umgibt und fest mit demselben verbunden ist, wobei die Spirale (16) proximale und distale Enden hat, dadurch gekennzeichnet, daß der Führungsdraht einen sich progressiv vermindernden Durchmesser hat, die Spirale (16) einen Durchmesser hat, der in Richtung des distalen Endes abnimmt, und daß die Spirale (16) aus einem Draht (17) ausgebildet ist, der einen Durchmesser hat, der von einem zum anderen Ende abnimmt und der zu einer Spirale gewunden ist, so daß der durchmessergrößere Abschnitt des Drahts in einem dem proximalen Ende näherliegenden Bereich beginnt und der durchmesserkleinere Abschnitt des Drahts in einem dem distalen Ende näherliegenden Bereich endet.

2. Führungsdraht nach Anspruch 1, der eine zweite Spirale (23) aufweist, die aus einem strahlenundurchlässigen Material ausgebildet ist und ein Ende hat, das an das distale Ende der ersten Spirale (16) angrenzt.

3. Führungsdraht nach Anspruch 2, dadurch gekennzeichnet, daß die zweite Spirale (23) einen im wesentlichen gleichförmigen Durchmesser hat und eine abgerundete Spitze (36) am distalen Ende vorgesehen ist.

4. Führungsdraht nach Anspruch 2 oder Anspruch 3, dadurch gekennzeichnet, daß die angrenzenden Enden der ersten und zweiten Spiralen (26, 23) zusammengeschraubt und haftend mit dem Kernelement (12) verbunden sind.

5. Führungsdraht nach einem der vorangehenden Ansprüche, der eine dritte Spirale (26) aufweist, die proximale und distale Enden hat, die von dem Kernelement (5) getragen werden und bei dem das distale Ende an das proximale Ende der ersten Spirale (16) angrenzt und eine Einrichtung (33) eine haftende Verbindung zwischen den angrenzenden Enden der ersten und dritten Spiralen und dem Kernelement bildet.

6. Führungsdraht nach Anspruch 5, dadurch gekennzeichnet, daß die dritte Spirale (26) mit einem Kunststoffüberzug bedeckt ist.

7. Führungsdraht nach Anspruch 5 oder Anspruch 6, der im Verbund einen Polyethylenschlauch (31) aufweist, der von dem proximalen Ende des Kernelements (12) getragen wird und der ein distales Ende hat, das an das proximale Ende der dritte Spirale (26) angrenzt.

8. Führungsdraht nach einem der vorangehenden Ansprüche, dadurch gekennzeichnet, daß das längliche Kernelement (12) zylindrische Abschnitte mit unterschiedlichen Durchmessern und konische Abschnitte mit unterschiedlichen Durchmessern hat, die an die zylindrischen Abschnitte angrenzen.

9. Führungsdraht nach einem der vorangehenden Ansprüche, dadurch gekennzeichnet, daß die erste Spirale (16) aus einem Draht (17) ausgebildet ist, der zylindrische Abschnitte mit unterschiedlichen Durchmessern hat, wobei konische Abschnitte mit unterschiedlichen Durchmessern an die zylindrischen Abschnitte angrenzen.

**Revendications**

1. Filet de guidage comprenant un élément central allongé (12), présentant des extrémités proximale et distale (13, 14) et une zone en coupe qui décroît en direction de l'extrémité distale, et une bobine de support (16), entourant l'élément central (12) et fixée à celuici, la bobine (16) présentant des extrémités proximale et distale, caractérisé en ce que le filet de guidage présente un diamètre progressivement atténué, la bobine (16) présente un diamètre qui décroît en direction de l'extrémité distale, et en ce que la bobine (16) est formée d'un filet (17) présentant un diamètre qui décroît d'une extrémité à l'autre et qui est enroulé de façon hélicoïdale, de sorte que la partie du filet ayant le plus grand diamètre commence dans une zone située à proximité de l'extrémité proximale et la partie du filet ayant le plus petit diamètre se termine dans une zone située à proximité de l'extrémité distale.

2. Filet de guidage selon la revendication 1, caractérisé en ce qu'il comprend une seconde bobine (23) formée d'un matériau radiopaque ayant une extrémité juxtaposée à l'extrémité distale de ladite première bobine (16).

3. Filet de guidage selon la revendication 2, caractérisé en ce que la seconde bobine (23) présente un diamètre substantiellement uniforme et une extrémité bombée (36) à son extrémité distale.

4. Filet de guidage selon l'une quelconque des revendications 2 et 3, caractérisé en ce que les extrémités juxtaposées de la première et de la seconde bobines (16, 23) sont vissées l'une à l'autre et sont fixées à l'élément central (12).

5. Filet de guidage selon l'une quelconque des revendications précédentes, caractérisé en ce qu'il comprend une troisième bobine (26) présentant des extrémités proximale et distale portées par l'élément central (12) et dont l'extrémité distale est juxtaposée à l'extrémité proximale de ladite première bobine (16) et des moyens (33) forment une fixation entre les extrémités juxtaposées de la première et de la troisième bobines et l'élément central.

6. Filet de guidage selon la revendication 5, caractérisé en ce que la troisième bobine (26) est recouverte d'une gaine plastique.

7. Filet de guidage selon l'une quelconque des revendications 5 et 6, caractérisé en ce qu'il comporte un tube (31) en polyéthylène qui est porté par l'extrémité proximale de l'élément central (12) et dont l'extrémité distale est juxtaposée à l'extrémité proximale de la troisième bobine (26).

8. Filet de guidage selon l'une quelconque des revendications précédentes, caractérisé en ce que l'élément central allongé (12) présente des parties cylindriques de différents diamètres et des parties effilées de différents diamètres, adjacentes aux parties cylindriques.

9. Filet de guidage selon l'une quelconque des revendications précédentes, caractérisé en ce que ladite première bobine (16) est formée d'un filet (17) présentant des parties cylindriques de différents diamètres avec des parties effilées de différents diamètres, adjacentes aux parties cylindriques.

EP 0 200 430 B1

FIG.—1

11

38

31

36 23

34

16 33 26

FIG.—2

FIG. —3

FIG. —4

FIG. —5

0.4572 mm
(.018 in)

0.6858mm
(.027in)

16

0.889mm
(.035in)

16c

16b

16a

1cm    8.5cm    6cm

## FIG. — 6

0.4572 mm MAX
(.018in MAX)

23

3cm ±1mm

## FIG. — 7

0.8636/0.9017mm
(.034/.0355in)
AFTER
PTFE COAT

26

81.5cm±1mm

## FIG. — 8

0.6604 mm
APPROX(.026 in)

31

45cm

(.041in)
10.414 mm

## FIG. — 9